# EUROPEAN PATENT APPLICATION

(11) **EP 2 093 684 A2**
(43) Date of publication of application: **26.08.2009**
(21) Application number: 09153545.0
(22) Date of filing: 24.02.2009
(51) Int. Cl.: G06F 19/00

(54) **Intelligent dashboards**

(30) Priority: 24.02.2008 US 36287
(71) Applicant: Karl Storz Endoscopy-America, Inc., Culver City, CA 90230 (US)
(72) Inventor: Martin, Neil A., Encino, CA 91436 (US); Buxey, Farzad D., Marina del Rey, CA 90292 (US); Zlatev, Vesselin, Aliso Viejo, CA 90230 (US)
(74) Representative: Witte, Weller & Partner

(57) **Abstract**

Some embodiments of the invention provide an intelligent method for displaying patient data. The method identifies a medical or non-medical condition. Based on the identified condition, the method then identifies a user interface for displaying patient data to a user.

## Description

### FIELD OF THE INVENTION

The invention is directed towards a clinical information system that provides intelligent dashboards for viewing patient data.

### BACKGROUND OF THE INVENTION

In recent years, hospitals have increased the amount of information they produce about each patient in digital form to an extent that would be overwhelming to a human being trying to cope with every bit of that information. For example, a patient's heart rate or blood pressure might be continuously monitored with a new value generated several times a minute.

Accordingly, systems for displaying such data have been developed. Some of these systems take the form of dashboards for computer or other electronic displays for displaying specific information about a patient. Unfortunately, in many cases, the overwhelming amount of raw data has been replaced by an overwhelming number of different options as to which dashboard will provide the most useful information about a patient at any given time. Therefore, a need has arisen for a system that helps a user select an appropriate dashboard to use to display information about a selected patient.

### SUMMARY OF THE INVENTION

Some embodiments of the invention provide an intelligent method for displaying patient data. The method identifies a situational condition relating to a patient or user of the system. Based on the identified condition, the method then identifies a user interface for displaying patient data to a user. In some embodiments, a condition could be a condition determined by a set of vital statistics, or by any other piece of information, or collection of information relating to the patient or the person viewing the dashboard. The condition could be whether the user viewing the patient data is a doctor or nurse, or what kind of doctor (e.g. neurologist or osteopath), what department the patient is in (e.g. ICU, cardiology ward, etc) or any other fact about the patient, the user, the location or anything else deemed relevant to selecting an appropriate dashboard or any combination of such variables.

In some embodiments, the method displays the user interface to the user automatically upon identification of the condition. In other embodiments, the method displays the identified user interface in a list of recommended user interfaces. In these embodiments, the user can then select the identified user interface from the recommended list in order to view the identified user interface. The method of some embodiments then receives a user selection of a user interface from the recommended list. The method then displays the selected user interface and patient information in the selected user interface according to parameters of the selected user interface. In other embodiments, the method automatically chooses a user interface based on an identified condition rather than presenting recommended user interfaces.

### BRIEF DESCRIPTION OF THE DRAWINGS

The novel features of the invention are set forth in the appended claims. However, for purpose of explanation, several embodiments of the invention are set forth in the following figures.

**Figure 1** illustrates the system of some embodiments.

**Figure 2** illustrates four different dashboards.

**Figures 3a-3b** illustrate a clinical information system (CIS) application user interface of some embodiments.

**Figure 4** illustrates an intelligent dashboard process.

**Figure 5** illustrates some components of the system in some embodiments.

**Figure 6** illustrates a graphical user interface for providing recommendations.

**Figure 7** illustrates a software block diagram of some embodiments.

**Figure 8** illustrates a rules editing process.

**Figure 9** illustrates a rules editor of some embodiments.

**Figure 10** illustrates a process of editing and saving a dashboard.

**Figure 11** illustrates a sequential modification of a dashboard.

**Figure 12** illustrates a modification of the dashboard of **Figure 3b****.**

**Figure 13** illustrates a table of permissions of some embodiments.

**Figure 14** illustrates an example of a recommendation list with a public dashboard and attribution.

**Figure 15** illustrates some components of the system in an alternate embodiment.

### DETAILED DESCRIPTION OF THE INVENTION

In the following description, numerous details are set forth for purpose of explanation. However, one of ordinary skill in the art will realize that the invention may be practiced without the use of these specific details. For instance, the techniques described below are described in a specified order, but other embodiments may change the order of the operations while still embodying the current invention.

Some embodiments of the invention provide an intelligent method for displaying patient data. The method identifies patient conditions (including non-medical conditions such as the primary physician's department or location of a user looking at information about the patient). Based on the identified condition, it then identifies a user interface for displaying patient data to a user.

In the discussion below, the term dashboard is used to refer to a user interface (UI) that is used for displaying patient data. In the example below, a dashboard is a collection of window panes, with each window pane providing one or more views of a set of patient data (e.g., patient clinical data). Several examples of dashboards are provided in Section II. Before providing these examples, Section I describes one environment for implementing the intelligent UI selection methodology of some embodiments of the invention.

After the providing several dashboard examples in Section II, Section III describes the intelligent dashboard selection methodology of some embodiments in further detail. In some embodiments, this methodology has a knowledge base and a library of dashboards that enhanced over time based on user feedback. Section IV then describes the knowledge base, the library of dashboards, and improvements to them based on user feedback.

**I. OVERVIEW**

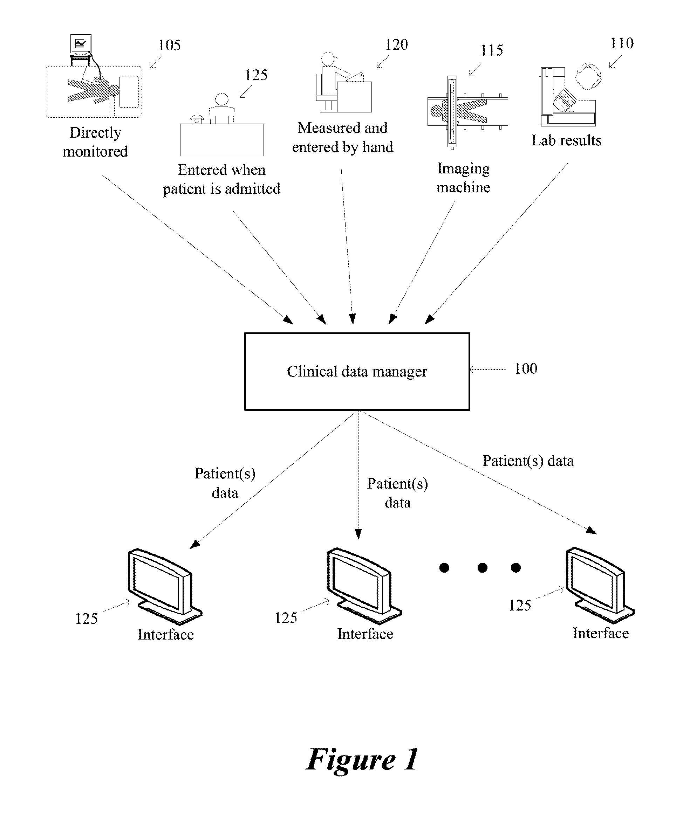

**Figure 1** illustrates a clinical data manager 100 in which some embodiments of the invention are implemented. The clinical data manager 100 collects patient data from various sources. In some embodiments, the patients may be in one unit of a hospital, in one hospital, or in several hospitals. As shown in **Figure 1****,** the collected data can come from a variety of sources, including sensors 105, lab tests 110, scans 115, recordings measured by medical personnel 120, information gathered when the patient is admitted and entered into an interface 125, or other sources of information about the patient or the patient's medical data.

The clinical data manager 100 receives, normalizes, analyzes, stores and/or aggregates the patient data. It performs these functions for the purposes of gathering data about individual patients, as a snapshot of a patient's data or as a record of the data over time. These operations also allow the system to compare statistics among patients (in some cases including the change in statistics of each patient) for various reasons, e.g., in order to efficiently allocate medical resources.

Through various interfaces 125, the clinical data manager 100 reports data, disseminates data, and/or issues alerts to users based on this data. In some embodiments, these interfaces are different from each other depending on the job of the user within the medical system (e.g. doctor or nurse, what kind of doctor, etc.), the particular location in which the interfaces are displayed (e.g. cardiac ICU or coma ward), and the momentary needs of the individual user and/or patient. In the illustrated embodiment of **Figure 1****,** an interface 125 can be used for entering patient data when the patient is admitted, in other embodiments, different systems are used for entering patient admission data and for viewing patient data.

As mentioned above, some embodiments provide a methodology for automatically selecting an interface from among several interfaces (or dashboards) based on various conditions. As further described below, the interface selected can also be based on data relating to the patient (including medical and non-medical data), the identity of the user viewing the patient data, the ward of the hospital in which the user is when he wants to view the patient's data and on other factors not directly related to the individual patient, but related to those trying to access the data about the patient or other circumstances surrounding the attempt to access the data (e.g. time of day, etc.). In some embodiments, the information gathered by medical personel 120 can be entered into an interface 125.

In some embodiments, the dashboard displays information that includes those data required to assess the severity of the condition, the trend (improving or deteriorating) of the condition, the cause of the condition, and/or the secondary consequences of the condition (e.g. on other organ systems). Furthermore, the dashboard provides the data that is required to determine an appropriate response. An appropriate response might include the ordering of additional lab tests or other diagnostic tests, ordering or changing medication, or scheduling invasive procedures or surgery.

In some embodiments, the dashboard displays information that includes established treatments, guidelines or protocols. The information may come from public reference sources or from customized intramural institutional policies. For instance, if the condition is hyperglycemia and the particular hospital has a policy for how to treat hyperglycemia, then a user can configure a dashboard to display that policy in a window of the dashboard. In some embodiments, the policy displayed in the dashboard is linked to a repository of policies, so when the policy is changed in the repository, the policy displayed when the dashboard is opened also changes.

In some embodiments, the information provided by an intelligent dashboard and the specific mode of display of the information in the intelligent dashboard are configured to answer the question "given this condition, what else would a health care provider (e.g. a nurse or doctor) need to see in order to fully assess the condition and respond appropriately?"

Accordingly, in some embodiments, the dashboard displays information where the information and the mode of displaying that information are specifically designed and configured with intent to follow the typical train of thought and sequence of assessment that a highly experienced expert clinician would follow, or to follow established best practices.

Where a prior art dashboard gives a menu of options that is the same no matter what (e.g. general categories of information). The intelligent dashboard of some embodiments decides what the most relevant data are and how to display them. Once the system identifies a condition the system brings up the information relevant to that condition quickly and easily (e.g. without needing to click several times each in several different windows to pull up the relevant data). Out of the massive array of data that a user could pull up from a myriad of menus, sub-menus and sub-sub-menus, the intelligent dashboard system pushes the appropriate data to the front in the manner most relevant to the user.

For instance, if a particular doctor wants to see certain types of information every time he logs in in the ICU. This information is automatically displayed in the various window panes of the intelligent dashboard. Rather than starting with a window that says "radiology" and has an entire list of scans, the dashboard knows that he wants to see today's and yesterday's chest x-ray. Accordingly, when a doctor selects a patient, all the info that the doctor wants is pushed forward in a dashboard.

**II. DASHBOARDS**

**A. Overview of dashboards**

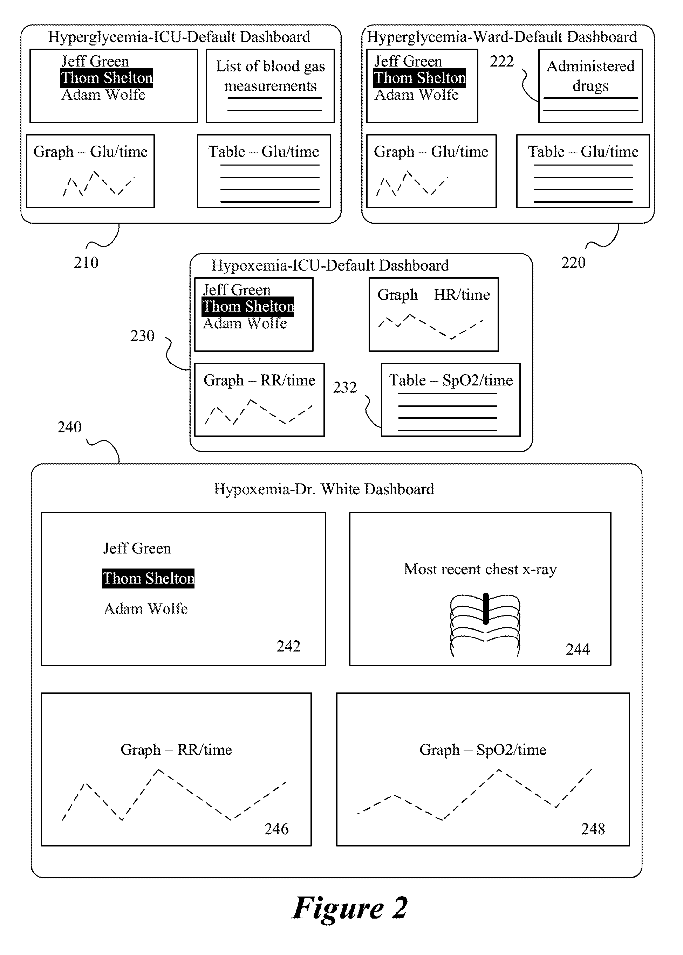

Various examples of the user interfaces (or dashboards) that could be recommended by some embodiments are illustrated in **Figure 2. Figure 2** illustrates four different dashboards 210-240. The systems of various embodiments display dashboards on a variety of interface devices in a variety of embodiments, e.g. computer displays, PDAs, cell phones, etc. Dashboards 210 and 220 are alternate dashboards for displaying data relevant to a patient with hyperglycemia. Dashboards 230 and 240 are alternate dashboards for displaying data relevant to a patient with hypoxemia.

Each dashboard 210, 220, 230 and 240 includes multiple window panes, such as the window panes 222, 232, 242, 244, 246, and 248. The various window panes of the dashboards contain information about the selected patient. For instance, the window pane 222 shows a list of drugs administered to the patient (e.g. drugs, dosages, and times), the window pane 232 shows the percentage of oxygen saturation in blood (SpO2) in a table of measurements, the window pane 244 shows an image of a patient's most recent chest x-ray, the window pane 246 shows a graph of the patient's respiratory rate over time, and the window pane 248 shows a graph of the patient's SpO2 level over time.

In some embodiments, each dashboard includes a patient list window, such as the patient list window 242 of dashboard 240. The patient list window 242 provides a list of the patients, recorded clinical data regarding each patient, computed scores generated from patient clinical data, and trends associated with the recorded data and generated scores. In some embodiments, the patient list 242 is editable, selectable, or clickable. In other embodiments, the list of patient names is not considered part of the dashboard. In some embodiments, the individual dashboards are recommended to users based on the intelligent dashboard system described in section III below. Some intelligent dashboard systems use a user interface such as the one described in subsection II.B. below.

**B. Clinical Information System Application User Interface**

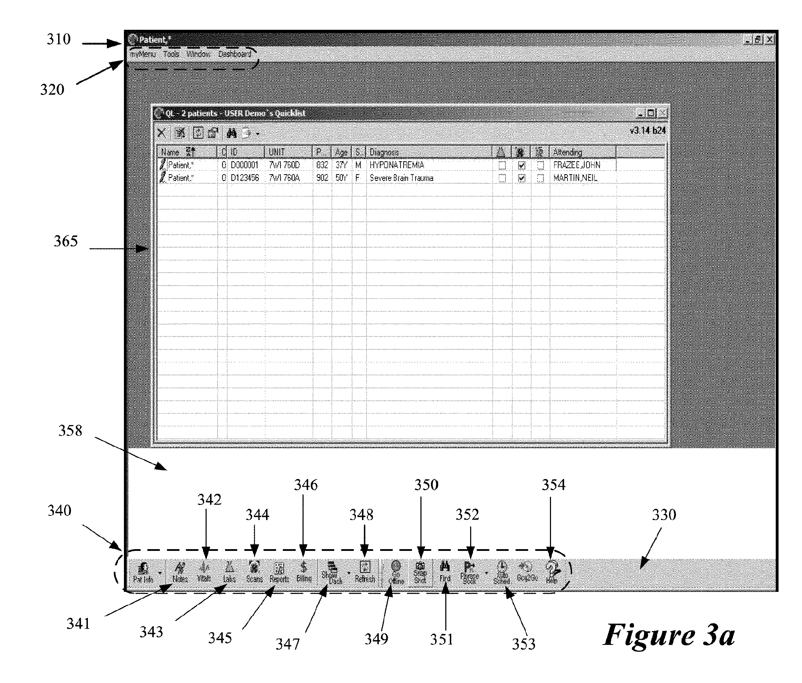

**Figures 3a-3b** illustrate a clinical information system (CIS) application user interface of some embodiments. In **Figure 3a****,** the user interface provides a master window 310 including a master window menu bar 320, master window toolbar 330, master window toolbar icons 340, master window viewing area 358, and patient list 365.

The master window 310 encloses the master window menu bar 320, master window toolbar 330, and master window viewing area 358. The master window menu bar 320 is located at the top of the CIS application user interface. The master window menu bar 320 lists available menu options for the CIS dashboard. When a menu bar option is selected (via a mouse click or appropriate keyboard sequence), the menu "pulls down", revealing a list of menu items or options. These options enable the user to perform various actions within the CIS dashboard. When working offline, some menu options are not available and are grayed out.

The master window toolbar 330 includes the master window toolbar icons 340. The master window toolbar 330 appears at the bottom of the CIS application and contains toolbar icons 340 to access CIS dashboard functionality. When one of the master window toolbar icons 340 is selected, the corresponding function appears in the master window viewing area 358.

Available master window toolbar icons 340 in the master window toolbar 330 include a notes icon 341, a vital signs icon 342, a clinical labs icon 343, a scans icon 344, a reports icon 345, a billing icon 346, a show dashboard icon 347, a refresh icon 348, an applications icon (not shown), a go offline icon 349, a snap shot icon 350, a find icon 351, a phrase book icon 352, an auto schedule icon 353, and a help icon 354.

The notes icon 341 opens a new window pane that allows the user to enter clinical information into data entry forms or notes. The user can select from an existing list of notes designed by health care professionals. Examples of notes in the CIS Dashboard include nursing notes and neurosurgery encounter notes. The default for this button is called the default note and is configured via a menu item.

The vital signs 342 icon opens a new window pane that displays the patients near real-time vital sign data as monitored and communicated by the patient monitor. Available data displays include but are not limited to (a) vital sign waveform data (i.e. multi-lead ECG, invasive blood pressure ART, PAP, CVP, etc., respiration, EtCO2, SpO2, CO), (b) trend data (i.e. line trends, tabular trend data), and (c) current vital parameters updated every few seconds.

The clinical labs icon 343 opens a new window pane that displays the patient's clinical lab data results as provided by the hospitals lab information system. Data views include but are not limited to (a) present day lab results, and (b) retrospective day-by-day lab results. Lab results are color coded into groups. Abnormally high values are highlighted in purple, low values are highlighted in blue, and normal values are not highlighted. A dashboard can display lab results in tabular format and line trends.

The scans icon 344 opens a new window pane that displays the patient's radiology images as provided by the PACS. Radiology data types include but are not limited to (a) X-ray images, (b) MRI scans, (c) CT scans, (d) PET scans, (e) Dynamic Images (Cine Mode) and (f) Echo Cardiac Ultrasound. The CIS medical image application program provides a standard PAC image viewer with the ability to manipulate images (i.e. zoom, rotate, pan, contrast, inversion).

The reports icon 345 opens a new window pane that displays a list of patient specific reports. These include but are not limited to scanned text records, orders, and reports in PDF format. The billing icon 346 opens a new window pane that displays the user-defined form (e.g., a neurosurgery encounter form). The default for this button is called the charge capture form and is configured via the menu item. The show dashboard icon 347 reloads the default configuration of dashboard windows in the viewing area. The pull-down arrow displays a listing of available dashboard configurations for selection.

The refresh icon 348 allows the user to manually reload or update the patient data presented in the CIS dashboard. The applications icon (not shown) opens a new window pane that allows the user to open an external application (e.g., a drug reference database) to the CIS dashboard. The external application runs in a separate window on the user's computer.

Similarly, a web icon (not shown) opens a new window pane that allows a user to browse the web within that pane. A user can set a window pane to a specific URL and save the setting along with the rest of the dashboard. For example, a dashboard can pull up a URL in a web pane when the dashboard is loaded that is either on an intranet or the Internet and shows information useful for treatment of a heart attack (e.g. that presents protocols for such treatment). This permits a dashboard to provide conditionally specific reference material (e.g. from a digital library).

The go offline icon 349 allows the user to toggle the state of the application from online state to offline state and back without logging in and logging off. The snap shot icon 350 allows the user to capture and save the information on the screen. The user can select to capture the full screen or only the active window.

The find icon 351 allows the user to search and locate one or more patient based on user-specific criteria. The selected patients can then be added to a quick reference list. The phrase book 352 icon allows the user to enter commonly used phrases when entering patient data into notes. The phrases are created and saved by the user and available in all text forms involving editing.

The auto schedule 353 icon allows the user to set automatic patient data downloads to the computer or handheld device activated at a user-defined schedule. The help icon 354 displays online help, which provides assistance in the use of the application.

Toolbar buttons 340 are different in different embodiments. Depending upon the configuration of a CIS, some of the application buttons may not be loaded on the interface. In some embodiments, some menu options are not available and are grayed out when a user is using the interface offline.

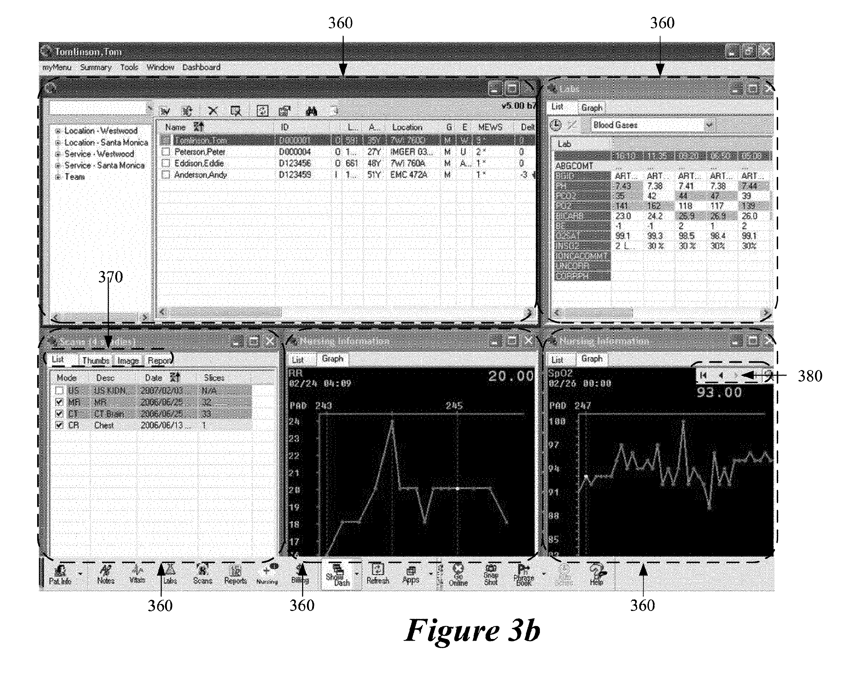

The master window viewing area 358 is the main area of the CIS dashboard that displays a patient list 365 containing patient information from various other hospital systems. In some embodiments, the master window viewing area 358 includes smaller windows called window panes. For instance, in Figure 3b, there are multiple window panes 360 displayed in the viewing area 358. Each of the window panes 360 can be arranged, resized, or managed by the user. In some embodiments, a user can click within the pane to modify data, sort data, copy, paste, or drag and drop data. The set of window panes 360 collectively comprise a CIS dashboard of the illustrated embodiment.

The window panes 360 are displayed in the master window viewing area of the CIS dashboard and present patient information collected and integrated from a variety of clinical systems. Each of the window panes 360 includes a set of selectable tabs 370, additional window pane toolbars, and controls 380.

The clinical data content of a window pane can be called a window pane "view". Some window panes are capable of displaying more than one different view. In some embodiments, selectable tabs 370 affect what view a window pane displays. For example, the set of selectable tabs 370 at the top of a window pane allow a user to select different views presenting different clinical data. A single view can have additional window pane toolbars and controls 380 to sort and navigate the clinical data presented. In some embodiments, such a CIS system includes an intelligent dashboard system for providing suggestions of dashboards to a user.

**III. INTELLIGENT DASHBOARD SYSTEM**

**A. Overview of intelligent dashboard system**

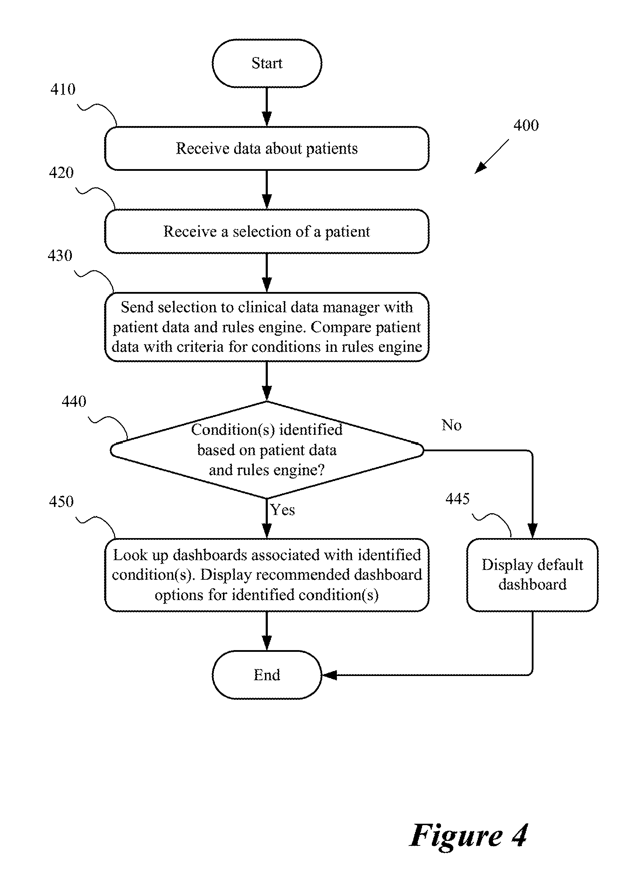

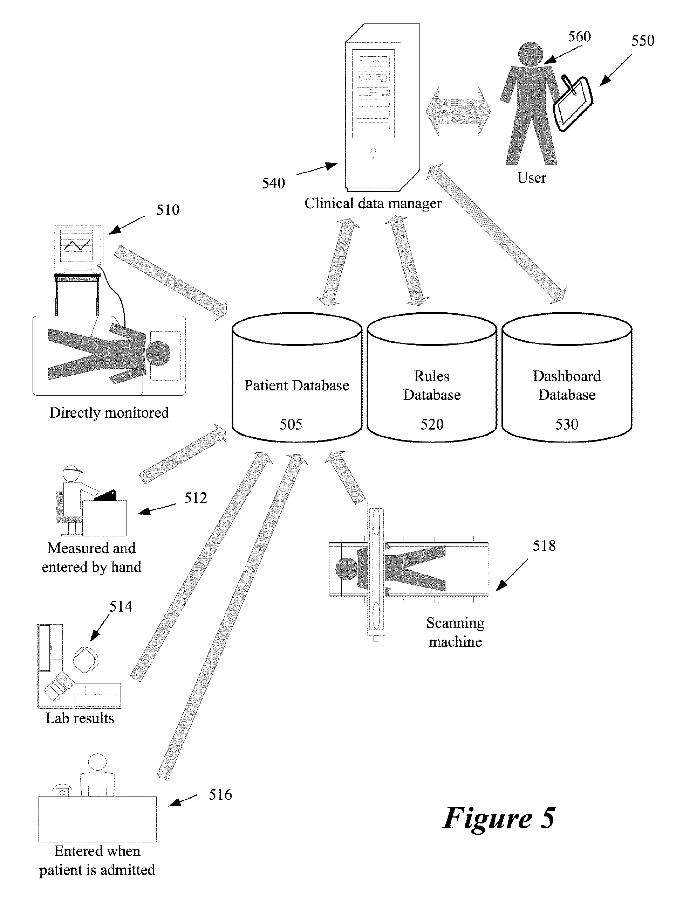

**Figure 4** illustrates an intelligent dashboard process 400 for identifying conditions and, based on the conditions, recommending dashboards to display patient statistics. The operations of **Figure 4** will be described in conjunction with **Figure 5****,** which illustrates some components of the system in some embodiments. One of ordinary skill in the art will realize that other embodiments may use different components than those illustrated in **Figure 5** while still remaining within the scope of the invention.

In operation 410, the process 400 receives patient data. As **Figure 5** shows, operation 410 can receive data from several different sources 510-518 (further described in subsection III.B. below) that feed data into a patient database 505. The process receives, at 420 a selection of a patient from display unit 550 by user 560.

Operation 430 sends the selection to a clinical data manager 540 with access to the patient data, a rules database 520, and a dashboard database 530. Operation 440 uses the clinical data manager 540 to analyze the patient data and compare various statistics about the patient to statistics that identify various conditions (e.g. medical conditions) in the rules database 520. In some embodiments, operation 440 can identify non-medical conditions. For example, the operation 440 could identify the condition that the patient's primary physician is a neurologist.

If the patient data does not match any of the conditions identified in the rules database 520, then operation 440 does not recommend a dashboard associated with an identified condition, a default dashboard is displayed by operation 445, and process 400 ends. In some embodiments, when no condition is identified, the process 400 recommends default dashboards and the user selects one, rather than the process 445 automatically displaying a default dashboard.

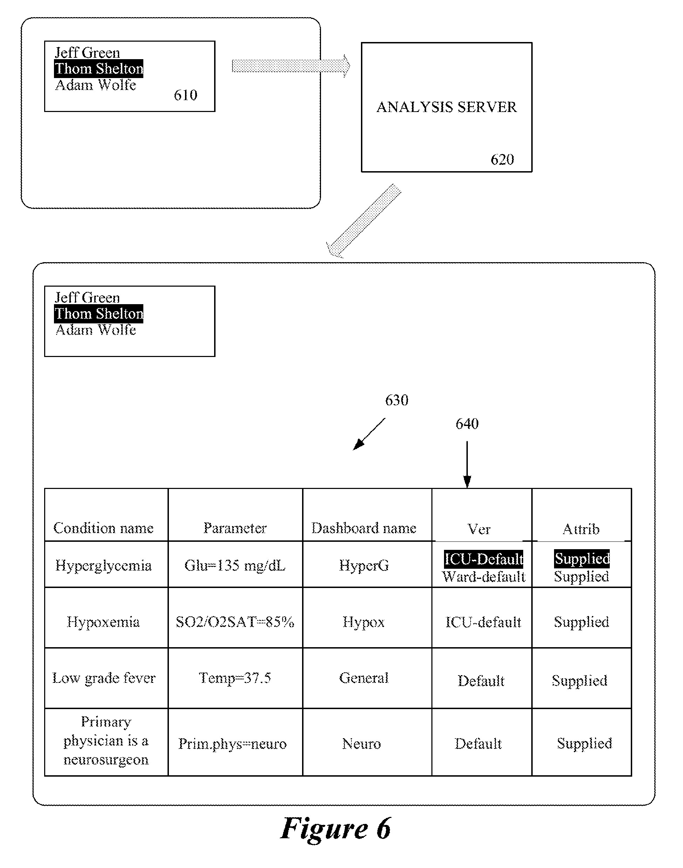

If operation 440 identifies at least one condition, then operation 450 looks up the dashboards associated with the identified condition(s) in the dashboard database 530 and presents them to the user 560 as options. A graphical user interface of some embodiments for performing operation 450 is illustrated in **Figure 6****.** After a patient's name is selected from list 610 and is passed to analysis server 620, and the analysis server 620 identifies conditions of the patient (as previously described in operations 420-440), a list of recommended dashboards 630 is displayed.

The list 630 of some embodiments contains the names of the conditions that the system has identified for the patient. For each of these condition names, the list shows the variable or variables that caused the system to identify the condition, along with the value of those variables exhibited by the patient. For example, hyperglycemia is identified by the glucose level and the patient's glucose level is 135 mg/dL. The list 630 also shows a general dashboard name and lists available versions of the dashboard 640. The list shows an attribution for each of the versions. In list 630, all of these attributions are "supplied" to indicate that the recommended dashboards are the dashboards supplied by the company that produced the intelligent dashboard application. Examples of other attributions are described in subsection IV.C. below. In some embodiments, each version of the dashboard has a different name and the system identifies all the available names rather than general names and versions as shown in **Figure 6****.**

One of ordinary skill in the art will realize that many of the specific features described above can be provided in different ways while remaining within the scope of the invention. For example, in some embodiments, two or more of the described databases may be combined into one database (e.g. a combined rules and dashboard or rules, patient data, and dashboard database). In some embodiments, a selection of an appropriate dashboard for a given condition may be made automatically for some or all users, rather than presenting the user with options, particularly if only one condition is identified and only one dashboard exists for that condition.

In some embodiments, the recommended list is a pop-up, superimposed on the dashboard. In other embodiments, the recommended list is an ordinary pane in the dashboard. In some embodiments, a list of identified conditions is provided; the user selects a condition; and only then are the available dashboards for the selected condition supplied. In some embodiments, a default dashboard is supplied while the system is waiting for the user to select a dashboard from a list. In some embodiments, the default dashboard is selected if the user does not make a selection in some pre-set amount of time.

In some embodiments, the recommendation list ranks conditions by the severity of the conditions, e.g., how abnormal the conditions are. That is, what the difference is in percentage between the values that triggered that condition and the upper or lower level of the condition threshold. For example, if the glucose is 300% above normal and the hypoxic percentage is only 20% off, then the recommendation list might list them in that order. In some embodiments, the recommendation list ranks conditions by the rate at which the severity is increasing. Further examples of determination of severity can be found in sub-section IV.B. below.

**B. Patient data sources**

As mentioned above, in some embodiments illustrated in **Figure 5****,** the patient database 505 receives data from a variety of sources. Direct monitoring sources 510 continuously keep track of some bit of information about the patient, for example heart-rate monitors, electrocardiographs, intracranial pressure monitors, etc. Some sources 512 are measured and entered into a computer manually, for example, blood pressure taken with an analog pressure cuff, weight, or direct observations such as "hives", "jaundice", etc. Lab results 514 can either be entered by hand (e.g. "sickle cell trait") or in some cases directly supplied to the system by the machines measuring the relative quantities (e.g. blood glucose 130 mg/dL). Some data in the database may be entered when the patient is admitted, some of the information from such sources may be non-medical, such as the name or ID number of the patient, the name of the patient's doctor, or insurance company, a number used for a system for tracking patient's within the hospital such as bar coded bracelets etc. Data such as images from medical scanners 518 can also be entered into the patient database. For example, digitized images of x-rays, CT scans, or MRIs.

**C. Software architecture**

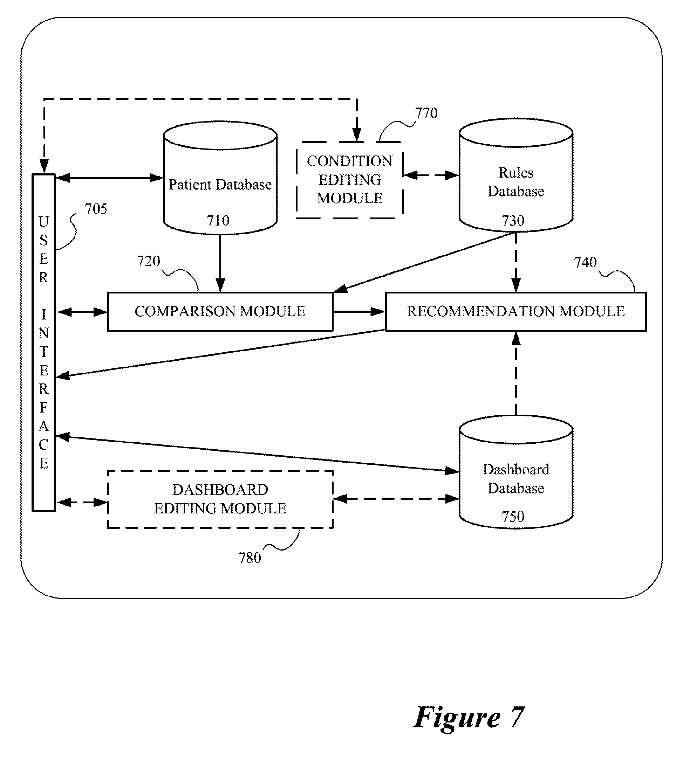

**Figure 7** illustrates a software block diagram of some embodiments for implementing process 400. A user interface 705 accesses patient database 710 to get a list of patient names and/or patient identification numbers. The user interface 705 is used to select a patient from the list of patients. The user interface 705 activates a comparison module 720 (sometimes called a "rules engine") that accesses data from the patient database 710 and a rules database 730. The comparison module 720 compares the data from the patient database 710 against the rules in the rules database 730 to determine whether the patient has any conditions identified in the rules database 730. In some embodiments, user information (e.g. ID, location, job) are provided by the user interface 705 for use in determining conditions. In other embodiments, user information is stored in the patient database 710. In other embodiments, some other source provides user information that is relevant to determining conditions.

If the patient does have any conditions identified in the rules database 730, the comparison module activates a recommendations module 740. The recommendations module 740 generates a list of the condition(s) of the patient along with a list of dashboards associated with the condition(s) and sends the lists to the interface module 705. In some embodiments, the recommendations module 740 receives the patient's condition(s) from the comparison module 720, and retrieves a list of dashboards associated with each condition from the rules database 730. In other embodiments, the recommendation module 740 receives the list of conditions and the lists of associated dashboards from the comparison module 720. In still other embodiments, the recommendation module 740 retrieves the lists of dashboards associated with each condition from a dashboard database 750.

In addition to identifying conditions from a database of rules and conditions and recommending dashboards from a database of dashboards, some embodiments allow users to edit the rules and conditions and/or dashboards in their respective databases. Accordingly, some embodiments include an editing module 770 that can be accessed by the user interface 705 to edit the rules and conditions in the rules database 730, examples of such embodiments are described in subsection IV.A. below. Also, some embodiments include an editing module 780 for allowing users to edit the dashboards, examples of such embodiments are described in subsection IV.C. below.

**IV. EDITING THE KNOWLEDGE BASE**

**A. Process for editing the rules**

As described above, the system of some embodiments has a rules database that identifies conditions. Such a rules database can include any number of saved rules/conditions, such as "if glucose > 130 mg/dL then patient has hyperglycemia". The rules (e.g. "if glucose>130 mg/dL) tie certain values of variables describing the patient's characteristics to identified conditions (e.g. hyperglycemia) in the database. In some embodiments, a user or organization can develop a rules database from scratch, add new conditions to an existing database, or amend the rules identifying existing conditions as needed.

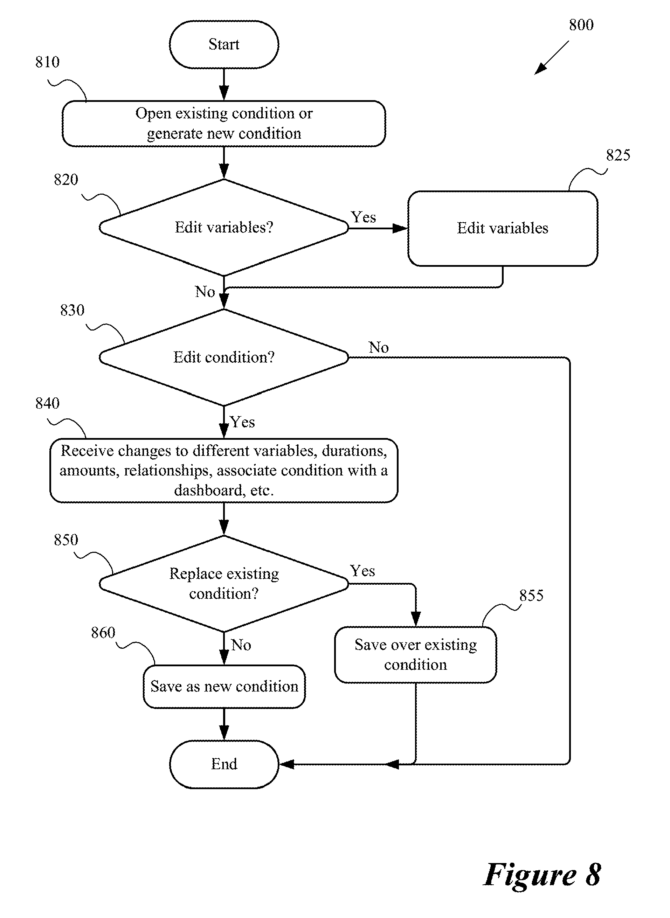

In some embodiments, users are able to edit the conditions that the comparison module (or rules engine) recognizes. The rules editing module 770 uses a rules editing process such as the one illustrated in **Figure 8** to teach the rules engine to recognize certain combinations of data as indicating particular conditions. The process 800 may use a rules editor, such as the one described in subsection IV.B, below.

Operation 810 starts the editing by opening an existing condition for editing or generating a new (e.g. blank) condition for editing. In some embodiments, the process includes an option 820 to edit variables in operation 830. Editing variables can include adding new variables (e.g. when a test is developed for a previously unknown or unmeasured substance in the blood) and changing the source from which the system will accept values of existing variables (e.g. when a hospital changes from using analog pressure measurements entered by hand to digital pressure measurements uploaded automatically). In some embodiments, a user can edit variables without first opening a condition for editing.

Once editing of the variables (if any) is concluded, the process has an option 830 to edit conditions. If the user does not want to edit the condition, then the process 800 terminates. If the user does want to edit the condition, then operation 840 receives changes to the variables, durations, amounts, and/or relationships that identify the condition, such changes are further described in subsection IV.B below.

After a condition has been edited, the process 800 has an option 850 for replacing an existing condition by saving over it at 855 (e.g. replacing the definition of hyperglycemia, by changing the threshold from 130 mg/dL to 120 mg/dL) or saving a new condition at 860 (e.g. adding a newly developed aggregate diagnostic score such as Multi Automated Severity Scoring to the previously existing conditions in the database).

One of ordinary skill in the art will realize that while the above description describes editing conditions discretely, some embodiments may provide a user with an entire list of conditions and their identifying rules and allowing changes to be made to any condition in the list before resaving the list.

**B. Rules editor**

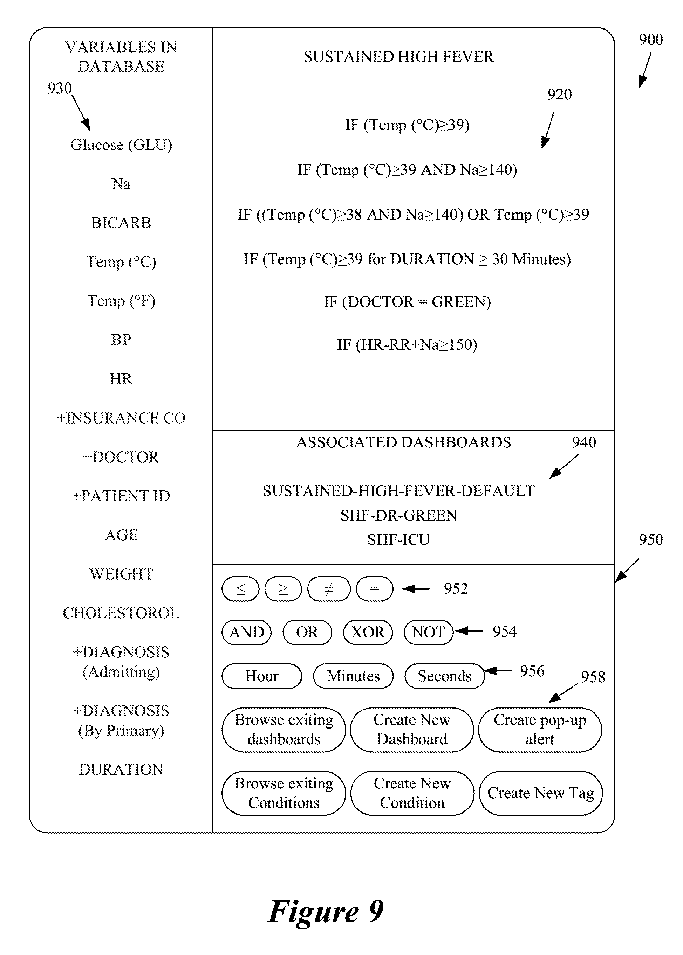

**Figure 9** illustrates a rules editor 900 of some embodiments. The editor contains a rules area that lists the condition 910 being edited and several possible rules 920 that define that condition. The editor contains a variables list 930 that lists the variables available for using to generate rules. The editor 900 contains an area 940 that lists the associated dashboards for the condition. The editor contains a set of buttons 950 for editing expressions in the rules 920.

In some embodiments, multiple sets of rules may indicate a single condition, however the multiple illustrated rules 920 are offered as multiple examples of possible rules. The rules 920 can include relational conditions (e.g. greater than, less than, greater than or equal to, etc.), such as "temperature greater than or equal to thirty-nine degrees". They can also include multiple conditions, such as "temperature greater than or equal to thirty-nine degrees AND sodium greater than or equal to 140 mmol/L". The rules can also include complex Boolean conditions, such as "(temperature greater than or equal to thirty-eight AND sodium greater than or equal to 140 mmol/L) OR temperature greater than or equal to thirty-nine". The rules can contain a duration associated with other variables, such as "temperature greater than or equal to thirty-nine for more than thirty minutes". The rules can even include mathematical formulae, such as "heart rate minus respiratory rate plus sodium is greater than or equal to 150". The rules can also be as simple as "Diagnosis (admitting)=Heart attack".

The variable list 930 could include any variables deemed necessary or relevant to determining conditions. In some embodiments, the variables relate to medical conditions only, however, in other embodiments, the variables could include such information as the patient's name, the patient's doctor, the type of doctor (e.g. neurologist), the patient's insurance company, the patient's ID number, whether the patient is scheduled for surgery, or discharge, or any other variables that whatever user has editing privileges for the list would enter into the list.

For example, individual doctors may have dashboards that they prefer to be used whenever one of their patients is examined. Names of doctors in the illustrated embodiment are preset into the system (as indicated by the "plus" sign next to the variable). Accordingly, when that variable is selected, the user can select a doctor from an available list (or add a new doctor to the list). In other embodiments, the doctor's name may be entered by hand, rather than from a list. Similarly, a particular patient may have some set of conditions that would be better monitored by a custom designed dashboard, rather than a general dashboard applicable to multiple patients. Accordingly, in some embodiments, the condition "This is patient Fred Smith" (or a unique patient ID number to avoid conflating same-name patients) may be defined with the rule "If patient=Fred Smith" and associated with a dashboard "FRED-SMITH-DASHBOARD".

The variables can also include variables do not relate directly to the patient. For example, the variables could be used to create patient conditions that depend on factors about the user who will be presented with the dashboards. For example, a "user-position" variable could check for values such as "nurse" or "doctor". Such a variable could also check for more specific values such as "cardiothoracic surgeon" or "podiatrist". Such variables could be used in rules to provide different dashboards for different personnel. In some embodiments, a variable for the location of the user could be used to identify a condition. For example, if the user is in an operating room, in an ICU or in an office.

By creating rules that combine various variables, a user can define very specific patient conditions. For example, a user can define a patient condition that only occurs when a patient has glucose above 135, the patient is in the cardiac unit, the patient's primary physician is a immunologist, the user trying to view data on the patient is an internist, the patient was diagnosed as having iron poisoning when admitted and the patient is scheduled for surgery in more than 48 hours but less than 72.

The list of dashboards in area 940 shows which dashboards are associated with the particular condition being edited, and thus, which dashboards will be offered as suggested dashboards when a patient is identified with variable values matching the rules for that condition. In some embodiments, the dashboards may have some identifying feature that indicates what classes of users or what individual users are authorized to use them. In some embodiments, the rules editor 900 can set permissions for the listed dashboards. More information on permissions can be found in subsection IV.C. below.

The set of buttons 950 includes buttons 952 for entering a relationship, such as "greater than or equal to". Buttons 954 allow a user to enter a Boolean condition (e.g. "AND" or "OR"). Buttons 956 allow users to set whether the duration for a condition should be measured in seconds, minutes, or hours. Buttons 958 allow a user to associate the condition being edited with an existing dashboard, create a new dashboard to associate with the condition, create an alert to pop up if the condition is detected, browse existing conditions, create a new condition or create a new variable as described in subsection IV.A above.

One of ordinary skill in the art will realize that the rules editor described above is merely an example and that rules editors with more, fewer, or different features could be used without departing from the scope of the present invention. For example, any of the associated buttons could be substituted with or used alternatively with hot-keys for activating the same functions.

In some alternate embodiments, conditions may include within their rules a gauge of the severity of the condition. This allows the experts who program the system to determine what conditions are most serious. This is useful when contrasting conditions for which a small deviation from the normal range is more significant than a large deviation in other conditions. For example, a 10% increase in body temperature over the normal 37 degrees Celsius (i.e. 40.7 degrees Celsius, or 105.3 degrees Fahrenheit) is a far more severe condition than a 50% increase in cholesterol levels. A user can assign a base line severity to some conditions, such as a high severity to conditions that identify an imminent heart attack. A user can also assign a "severity" to non-medical conditions. For instance, a particular doctor may have a preferred dashboard that will be the default for him unless an extremely severe medical condition is identified.

**C. Process for editing dashboards**

As described above, the system of some embodiments has a dashboard database that provides dashboards for displaying patient data. Such a dashboards database can store any number of dashboards associated with any number of different conditions. Each dashboard displays information from the patient database in a way that is designed to be relevant to the particular condition with which the dashboard is associated.

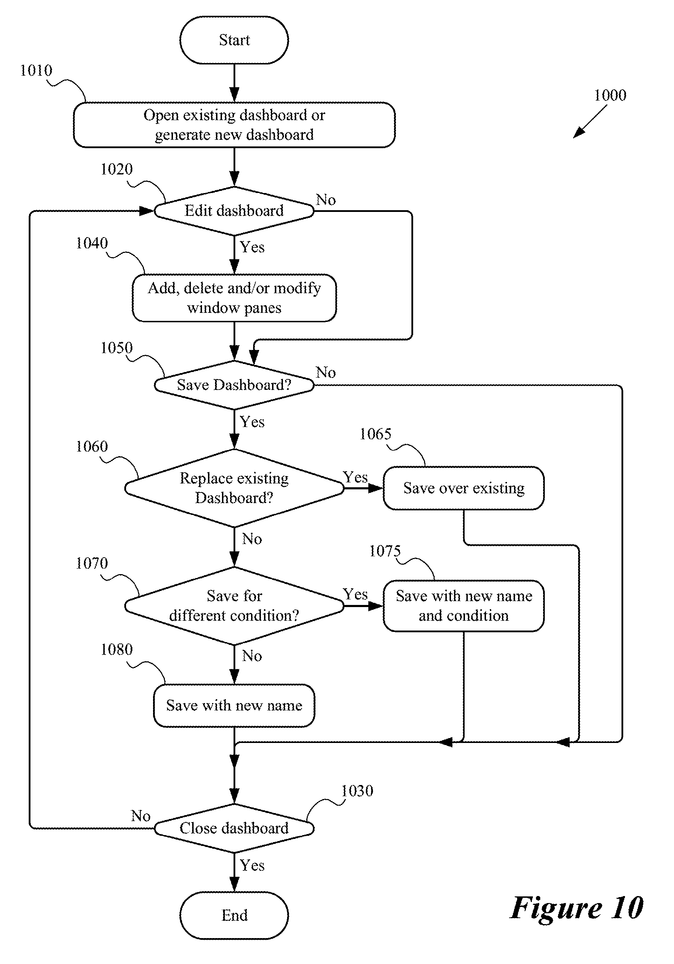

In some embodiments, users can modify dashboards and save their modifications so that when the condition associated with the dashboard is identified in another patient, the recommendation list includes a dashboard with the saved modifications, either instead of, or as well as the previous version. **Figure 10** illustrates a process 1000 of some embodiments for editing and saving a dashboard. The process 1000 starts with operation 1010, in which the process 1000 opens an existing dashboard or generates a new dashboard.

In some embodiments, opening a dashboard for editing is identical to opening a dashboard for use. This is reflected in option 1020 in which the system offers the user a choice of whether to edit the dashboard or not. In some embodiments, this is not offered as a direct confrontational choice, but instead, editing is an option as long as the dashboard is open. This is reflected in the loop between the editing option 1020 and the close dashboard option 1030 (after many intervening options and/or operations).

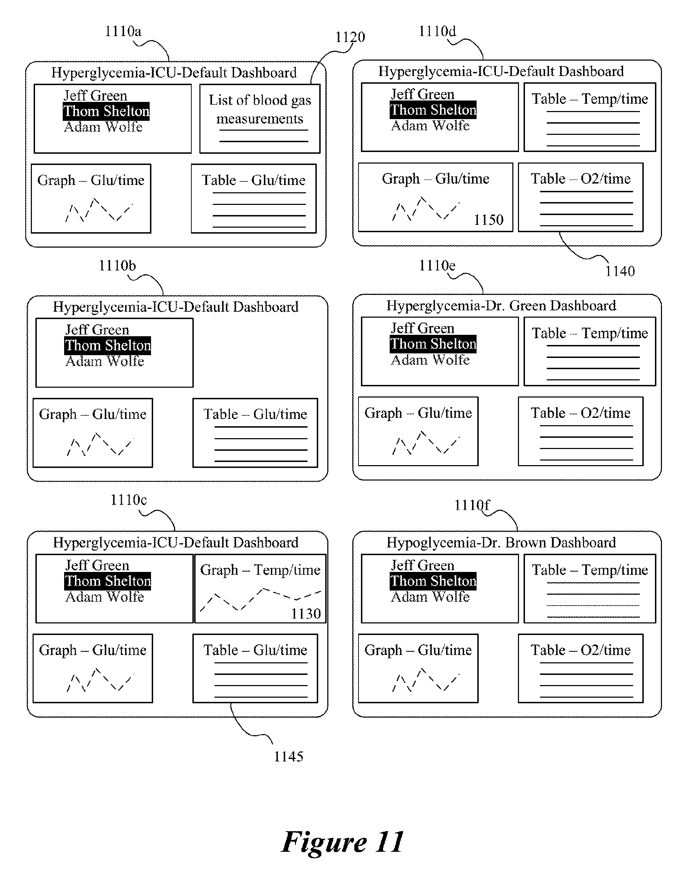

If the process receives commands from the user to edit the dashboard, then operation 1040 edits the dashboard according to the user's commands. Some modifications of dashboards are illustrated in the sequential dashboards of **Figure 11****.**

Dashboards 1110a-1110d represent different stages of editing a dashboard that illustrate the editing options of deleting, adding, and modifying window panes, while dashboards 1110e represent various saving options to be explained later. Dashboard 1110a shows a default dashboard including window pane 1120 that displays a list of blood gas measurements. In Dashboard 1110b the process 1040 has deleted window pane 1120 at the command of the user. In Dashboard 1110c, the operation 1040 has added a new window pane 1130 that displays a graph of temperature versus time. In Dashboard 1110c, the operation 1040 has modified pane 1130 so that it shows temperature versus time as a table, rather than as a graph. Dashboard 1110d contains window pane 1140 that shows 02 versus time rather than glucose versus time like the corresponding window pane 1145 in dashboard 1110c. Finally, in dashboard 1110c, the process 1040 has expanded the size of window pane 1150.

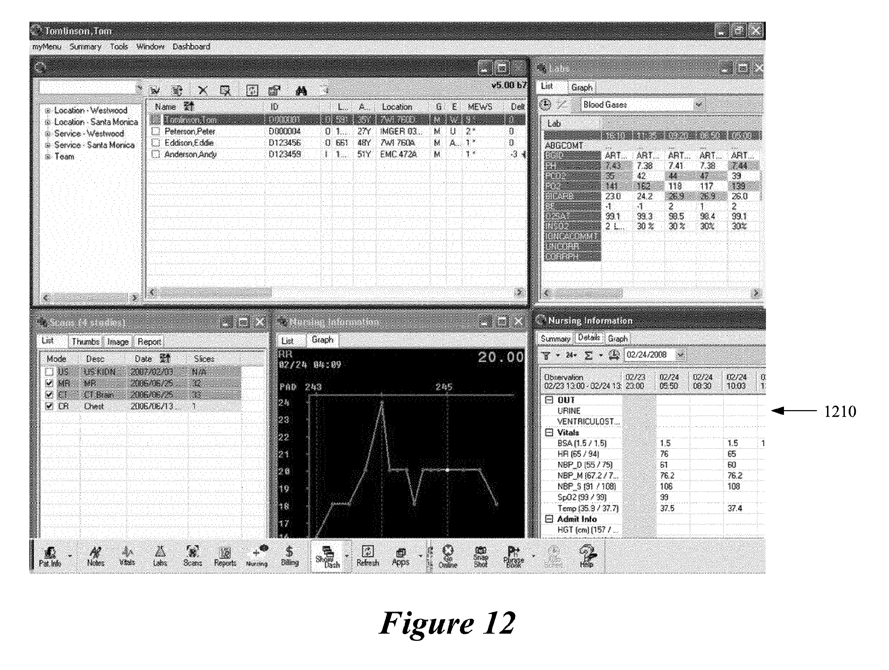

**Figure 12** illustrates an example of a modified dashboard from an exemplary application. The dashboard of window panes 360 from **Figure 3b** above is the default dashboard. A user has modified the dashboard to view the SpO2 data in a table 1210 with other vital statistics instead of as a graph.

Once the process 1000 has edited the dashboard in operation 1040, the process 1000 provides the user with the option to save the edited dashboard. If the user chooses not to save, then the process 1000 resumes the loop from 1020 to 1030. This allows the user to change a dashboard temporarily without saving a dashboard that the user wants to use but not save. If the user chooses to save, then the process 1000 offers option 1060, to replace the existing dashboard or not. If the user chooses to replace an existing dashboard, then operation 1065 saves the edited dashboard over the existing dashboard.

The process 1000 also offers the option 1070 to associate the dashboard with a different condition upon saving. If the user chooses option 1070, then the operation 1075 saves the dashboard under its new name for its new condition. **Figure 11** illustrates this in dashboard 111 Of in which the dashboard has been associated with hypoglycemia rather than hyperglycemia. This option is useful when several similar or related conditions exist which call for similar dashboards.

If the process 1000 is saving, is not replacing the existing dashboard, and is not associating the dashboard with a new condition then by process of elimination, the only option left is saving the dashboard for the existing condition, but under a new name. After all of the save options described above, the process 1000 proceeds to the close dashboard option 1030 and either closes the dashboard or returns to the options loop, starting with edit dashboard option 1120.

One of ordinary skill in the art will realize that the illustrated embodiment of data editing is only one possible embodiment of dashboard editing. Other embodiments are described in a U.S. Patent Application Serial No. 12/036,281, filed concurrently with this application. Said application is incorporated herein by reference. Even beyond that application, other embodiments are possible within the scope of the present invention.

**D. Security and permissions**

The previous parts of section IV describe the processes of editing conditions and dashboards. In order to keep the descriptions as simple as possible, the sections simply referred to "users" editing these items. However, in some embodiments, not all users have equal permissions to modify dashboards and conditions. In some instances, individuals may have private dashboards that others are not allowed to access. In others instances, in order to ensure that the default version is not lost, however users modify copies of it, the default version of a dashboard may be accessible to all, but can only be overwritten by a system administrator.

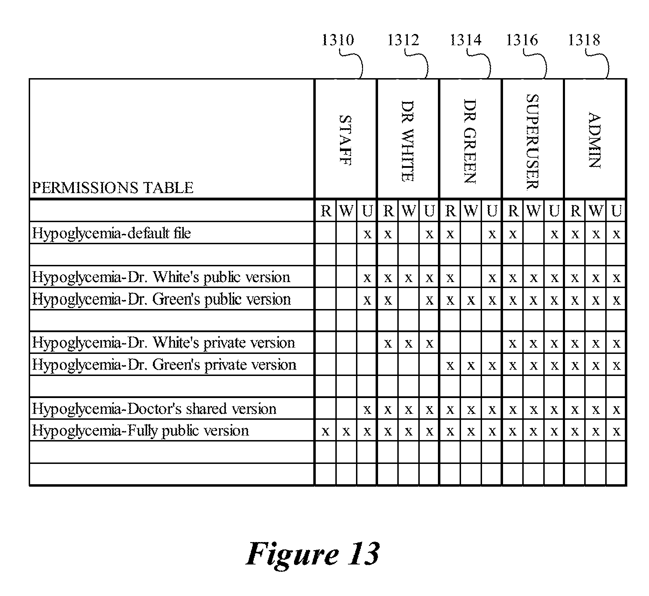

**Figure 13** illustrates a table of permissions of some embodiments for various dashboard files. The table identifies five users in four classes: a staff member 1310, doctors 1312 and 1314, a superuser 1316, and a system administrator 1318. Each of these users has different permissions for different files. If there is an "x" in the "W" column for a particular file, for a particular user, then the user has permission to write (e.g. overwrite or replace) the file. If there is an "x" in the "R" column for a particular file, for a particular user, then the user has permission to read (e.g. copy) the file. Reading a file can be useful, even if the user doesn't also have permission to write to the file. For example after reading a file, a user would be able to edit it and possibly save it under another name if they have write permission in any accessible file location. Finally, the last level of access is indicated by an "x" in the "U" column for a particular file, for a particular user. This indicates that the user has permission to use the file as a dashboard, even though they can't save it. In some embodiments, permission to use implies that the system allows the user to edit dashboards for one-time use of the modified dashboard, but not the ability to save the modified dashboard. The following description explains various levels of permission in the illustrated embodiment by explaining the typical permissions available at successively higher levels of access.

In the illustrated embodiment, the staff member 1310 has very low access rights. Other than a fully public file that is fully accessible to everyone, the staff member 1310 is not permitted by the system to read or write any file. The staff member 1310 can use only those dashboards designated for public use. This limited access is useful for staff members whose duties require them to monitor patients but who do not need to make their own dashboards.

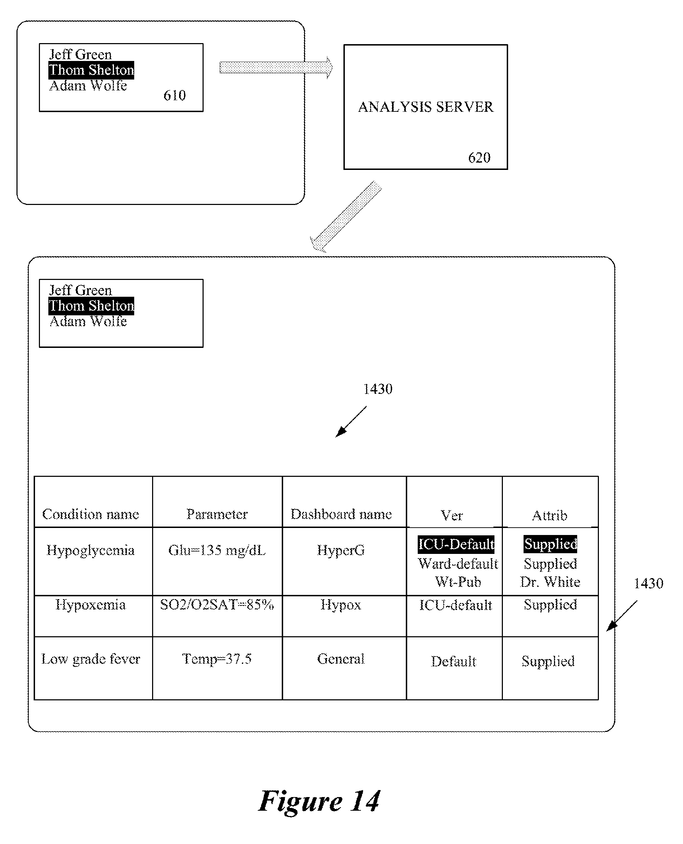

The doctors 1312 and 1314 can have private or public dashboards. Their public dashboards can be used by anyone, read by other doctors, and written by themselves and by the superuser 1316 and system administrator 1318. Having public versions available allows a doctor to make his own dashboard, let others benefit from the creation, but not have to worry about others deliberately or accidentally tampering with his work. **Figure 14** illustrates an example of a dashboard recommendation list 1430 with a doctor's public dashboard available for use and attributed to Dr. White.

The doctors' private dashboards are available only to themselves, superusers, and system administrators. This is useful as doctors may use dashboards privately that would confuse others. This also allows doctors to experiment with new dashboards without risking other users selecting an unfinished dashboard. Doctors can read the default dashboard but can't write to it. This prevents the default dashboard from being altered repeatedly by doctors with differing opinions of what should be in the default file.

A superuser 1316 is a user with higher than normal permissions, for example, a department head may need to access dashboards from any doctor in his department. In this embodiment, the higher than normal permission includes full access to doctors' public and private dashboards, but not permission to overwrite the default dashboard.

The system administrator 1318 of this embodiment has full access to all dashboards. This is useful because at least one user of the system is able to access everything and fix errors that may occur. For example, a system administrator 1318 can edit the default dashboards that need to be updated to reflect changes to the databases, new technology, or other reasons.

Appropriate entities can set these permissions. For example, the system administrator 1318 or a superuser 1316 can take particular dashboards out of use by removing all use permissions. In some embodiments, doctors determine who can use their versions, in others only superusers can allow anyone to use someone else's dashboards. This can be useful as a clutter prevention measure so that the staff isn't presented with 47 recommended versions from 47 different doctors. In some embodiments, only the creator of a dashboard can set permissions for it, even superusers or system administrators are unable to change it without permission. In some embodiments, the creator of a dashboard is the only one who can access it and there is no way within the normal functions of the system to give any other user access to it.

Similar restrictions on permissions apply to editing of conditions in some embodiments. In fact, there are good reasons to restrict permission to edit conditions more strictly than permission to edit dashboards. The rules database of some embodiments provides identification of patient conditions as well as recommending dashboards. If a dashboard does not contain information pertinent to an identified condition, a skilled medical practitioner is likely to recognize this and react accordingly, either by editing the dashboard or by switching to an alternate dashboard. However, if a condition is incorrect, a medical problem that could be easily treated may go unnoticed until it is too late. Accordingly, to reduce the risk of accidents, the rules engine of some embodiments is editable only by superusers, or even editable only by system administrators.

**V. ALTERNATE EMBODIMENTS**

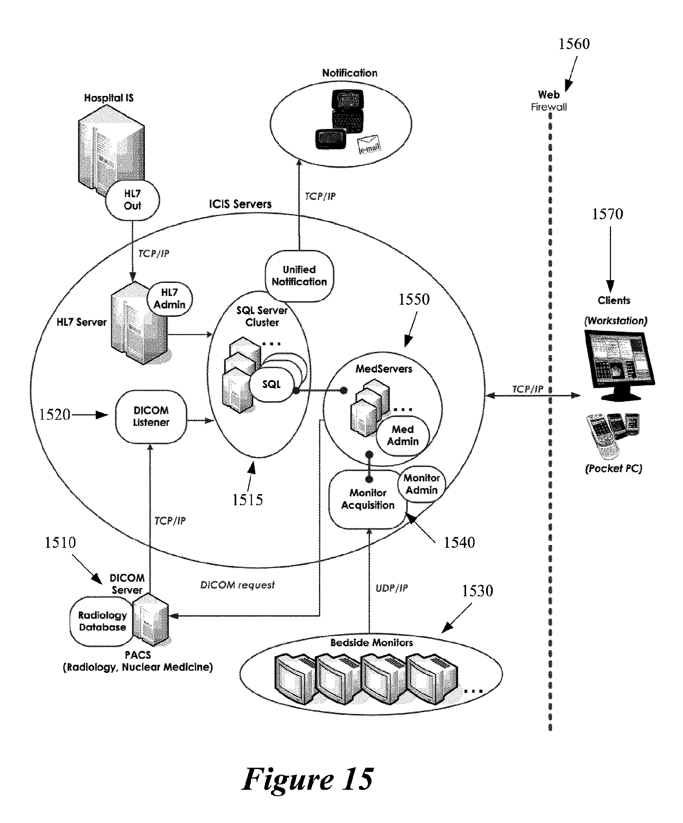

In some embodiments, such as the one illustrated in **Figure 15****,** the patient data is sent to the patient database through other servers, databases or subsystems, such as radiology database 1510, which stores radiology data (e.g. scanner images) and sends them to the patient database 1515 through DICOM listener 1520, or bedside monitors 1530 which send data to the patient database 1515 through monitor acquisition subsystem 1540 and medical servers 1550. **Figure 15** also illustrates that the system can send and receive data from outside the system (e.g. over the Internet) through a firewall 1560 to workstations and/or pocket PCs 1570.

One of ordinary skill in the art will realize that some embodiments exist as computer programs stored on computer readable media. Such computer programs contain sets of instructions that allow the program to perform the methods and provide the interfaces described in this application. One or more of the machines described in Figure 15 and in the figures described above may contain such computer readable media (e.g. memory, drives, etc.) and store programs with instructions to perform one or more operations of the processes described herein. Accordingly, a computer readable medium that contains such a program is within the scope of the present invention.

One of ordinary skill in the art will realize that some of the features described in this application are present in prior art (e.g. different permission levels for different users), however, they have not been used in combination with other features described herein. Furthermore, while the invention has been described with reference to numerous specific details, one of ordinary skill in the art will recognize that the invention can be embodied in other specific forms without departing from the spirit of the invention. For instance, alternate embodiments may allow more or fewer types of variables to affect recommendations. One of ordinary skill in the art would understand that the invention is not to be limited by the foregoing illustrative details, but rather is to be defined by the appended claims.
In other preferred embodiments of the present invention the method and/or the computer program stored on the computer readable storage medium has instructions for one or more of the following features:
- Providing controls for modifying the first dashboard and saving the modified dashboard;
- providing a set of security controls to limit user access,
- saving the modified dashboard;
- saving the modified dashboard with a flag that excludes at least one other user from using the modified dashboard;
- saving the modified dashboard with a flag that excludes at least one other user from making further modifications of the modified dashboard, but allows the user to use the modified dashboard;
- saving the modified dashboard with a flag that excludes at least one other user from saving further modifications of the modified dashboard, but allows said other user to use and modify the modified dashboard.
   In further preferred embodiments, which can be used either alone or in combination,
- saving the modifications comprises replacing the first dashboard with the modified dashboard;
- replacing the first dashboard requires authorization to replace the replace the first dashboard;
- the first dashboard comprises a window pane for displaying a table;
- the security controls limit a subset of users to using and modifying the plurality of dashboards without saving modifications;
- the security controls limit a subset of users to using the plurality of dashboards without modifying them;
- the rules engine is an external rules engine;
- the rules engine comprises rules received from a user authorized for identifying a condition;
- receiving a command to modify a rule in the rule engine is comprised;
- saving a modified rule is comprised.
   Further, it is pointed out that any number of instructions, be it one instruction or a combination of instructions, that are related to providing information to a user, in particular visual information, and/or accepting inputs from a user can be put into practice as instructions for implementing a graphical user interface (GUI) on a device.
   In one preferred embodiment a computer readable storage medium stores a computer program comprising sets of instructions for implementing a graphical user interface on a device, said GUI comprising:
   a) a patient selection list;
   b) a plurality of dashboards; and
   c) a list of at least one dashboard recommended for displaying a detected condition of a selected patient.
      In further preferred embodiments the GUI further comprises, alone or in combination,
- a control for adding a window pane to the first dashboard for displaying patient information not already displayed in the first dashboard;
- a control for saving a second dashboard that comprises the window panes of the first dashboard and the added window pane;
- controls for modifying the first dashboard and saving the modified dashboard;
- a set of security controls to limit user access;
- an alert when a user defined condition occurs;
- a set of controls for adding a new condition to a database and connecting the new condition to a recommended dashboard for that new condition.
   It is pointed out that any instructions for implementing a graphical user interface can be stored in a computer program on a computer readable storage medium. Further, any of the method steps disclosed can be represented, either alone or in combination, as instructions in a computer program. Also, each of the instructions comprised in a computer program can be represented as method steps in a method of displaying patient data.

## Claims

1. Computer readable storage medium storing a computer program for execution on one or more processors, said computer program comprising sets of instructions for:
a) receiving a selection of a patient;
b) identifying a condition of said patient based on medical data associated with said patient and a set of conditions in a database; and
c) displaying said condition and a recommendation of a dashboard relating to said condition for displaying medical data associated with said patient upon matching said medical data to a condition in said database.

2. Computer readable storage medium of claim 1, wherein a first dashboard of said plurality of dashboards comprises at least one window pane for displaying patient information relating to said condition.

3. Computer readable storage medium according to claim 1 or 2, further comprising instructions for providing a control for adding a window pane to said first dashboard for displaying patient information not already displayed in said first dashboard.

4. Computer readable storage medium according to any preceding claim, further comprising instructions for providing a control for saving a second dashboard that comprises the window panes of said first dashboard and said added window pane.

5. Computer readable storage medium according to any preceding claim, wherein said first dashboard comprises a window pane for displaying at least two variables as a graph, where in particular said two variables comprise time and a vital statistic.

6. Computer readable storage medium according to any preceding claim, wherein said list of at least one recommended dashboard comprises a plurality of dashboards based on different hospital departments.

7. Computer readable storage medium according to any preceding claim, further comprising instructions for providing an alert when a user defined condition occurs.

8. Computer readable storage medium according to any preceding claim, further comprising instructions for providing a set of controls for adding a new condition to a database and connecting said new condition to a recommended dashboard for that new condition.

9. Computer readable storage medium according to any preceding claim, wherein the instructions implement a graphical user interface (GUI) on a device, said GUI comprising:
a) a patient selection list;
b) a plurality of dashboards; and
c) a list of at least one dashboard recommended for displaying a detected condition of a selected patient.

10. Method of displaying patient data, in particular presented as instructions of a computer program stored on a computer readable storage medium according to any preceding claim, said method comprising:
a) identifying a patient condition based on a rules engine; and
b) identifying a dashboard for displaying patient information to a user.

11. Method according to claim 10 further comprising displaying said dashboard to the user automatically upon identification of said patient condition.

12. Method according to claim 10 or 11 further comprising listing said dashboard in a list of recommended dashboards.

13. Method according to any of claims 10 to 12 further comprising receiving a user selection of a dashboard of said list of recommended dashboards.

14. Method according to any of claims 10 to 13 further comprising displaying said selected dashboard and patient information in said selected dashboard according to parameters of said selected dashboard.

15. Method according to any of claims 10 to 14 further comprising receiving from said user a modification of said user selected dashboard, and modifying said dashboard accordingly.
